(19) Europäisches Patentamt
European Patent Office
Office européen des brevets

(11) Veröffentlichungsnummer: **0 134 499**
**B1**

(12) # EUROPÄISCHE PATENTSCHRIFT

(45) Veröffentlichungstag der Patentschrift: **25.02.87**

(51) Int. Cl.⁴: **C 07 C 87/40**, C 07 C 93/12, A 01 N 33/02

(21) Anmeldenummer: **84108205.0**

(22) Anmeldetag: **12.07.84**

(54) **Biscyclohexylaminalkane und Verfahren zur Bekämpfung von Pilzen.**

(30) Priorität: **19.07.83 DE 3325895**

(43) Veröffentlichungstag der Anmeldung: **20.03.85 Patentblatt 85/12**

(45) Bekanntmachung des Hinweises auf die Patenterteilung: **25.02.87 Patentblatt 87/9**

(84) Benannte Vertragsstaaten: **AT BE CH DE FR GB IT LI NL SE**

(56) Entgegenhaltungen:
**AT-B-334 872**
**DE-C-907 294**
**GB-A-1 550 051**
**US-A-2 606 928**

(73) Patentinhaber: **BASF Aktiengesellschaft, Carl-Bosch- Strasse 38, D-6700 Ludwigshafen (DE)**

(72) Erfinder: **Schuster, Ludwig, Dr., Weinheimer Strasse 44, D-6703 Limburgerhof (DE)**
Erfinder: **Ammermann, Eberhard, Dr., Sachsenstrasse 3, D-6700 Ludwigshafen (DE)**
Erfinder: **Pommer, Ernst- Heinrich, Dr., Berliner Platz, D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 134 499 B1

LIBER, STOCKHOLM 1987

**Beschreibung**

Die vorliegende Erfindung betrifft neue Biscyclohexylamine der allgemeinen Formel Ia

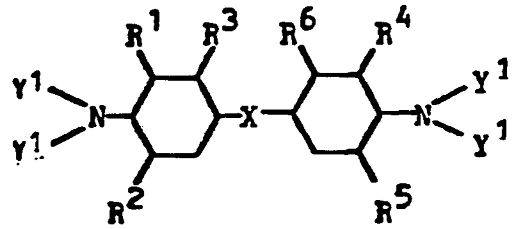

(Ia),

$R^1$, $R^2$, $R^4$ und $R^5$ gleich oder verschieden sind und Alkyl- oder Alkoxyreste mit 2 bis 5 C-Atomen je Rest, und $R^3$ und $R^6$ Wasserstoff bedeuten und $R^1$ und $R^3$ sowie $R^4$ und $R^6$ zusammen je einen an den Cyclohexylrest annellierten Cycloalkylrest bedeuten und $R^1$, $R^2$, $R^4$ und $R^5$ zusätzlich Wasserstoff bedeuten mit der Maßgabe, daß nur 2 der Reste $R^1$, $R^2$, $R^4$ und $R^5$ gleichzeitig Wasserstoff bedeuten, $R^3$ und $R^6$ zusätzlich Methoxy bedeuten mit der Maßgabe, daß 2 der Reste $R^1$, $R^2$, $R^4$ und $R^5$ Wasserstoff, X eine Einfachbindung, oder einen divalenten aliphatischen Rest mit 1 bis 8 C-Atomen, oder einen Cyclohexylenrest
und
Y einen Alkylrest mit einem bis vier C-Atomen bedeutet, wobei zwei Reste Y zusammen mit dem N zu einem Ring geschlossen sein können, außer der Verbindung in der $R^1$, $R^2$, $R^4$ und $R^5$ Isopropyl, $R^3$ und $R^6$ Wasserstoff, X Methylen und Y Wasserstoff bedeuten.

Weiterhin betrifft die Erfindung die Verwendung von Biscyclohexylaminen der allgemeinen Formel Ib

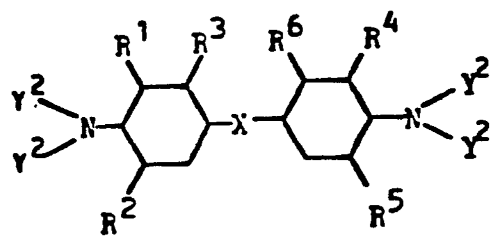

(Ib),

in der $Y^2$ Wasserstoff oder die Reste $Y^1$ bedeutet, als Fungizide, sowie fungizide Mittel, die Verbindungen der Formel Ib enthalten.

Aus der GB-PS 1 550 051 sind Verbindungen des Typs I', als Bausteine für Polyurethane bekannt.

Aufgabe der vorliegenden Erfindung war die Bereitstellung neuer und wirksamer Fungizide.

Demgemäß wurden die eingangs definierten Verbindungen Ia, sowie die Verwendung der eingangs definierten Verbindungen Ib als Fungizide und diese Verbindungen enthaltende fungizide Mittel gefunden.

Alkylreste mit 1 bis 5 C-Atomen sind beispielsweise Methyl, Ethyl, Propyl, Isopropyl, Butyl, sekundär Butyl, Isobutyl, Tertiärbutyl, Pentyl. Alkoxyreste mit 1 bis 5 C-Atomen sind beispielsweise Methoxy, Ethoxy, Propoxy, Isopropoxy. Annellierte Ccloalkylreste sind z.B. Methylenreste mit 3 - 5 C-Atomen, beispielsweise Trimethylen, Tetramethylen, Pentamethylen.

Die Biscyclohexylaminalkane treten als Mischungen verschiedener Stereoisomere auf. Üblicherweise werden diese Gemische nicht getrennt, sondern als Gemische Verwendet. Man kann jedoch - Vgl. Verbindungen Nr. 1 und 2 - durch Trennungsoperationen, beispielsweise durch Umkristallisieren - einzelne Isomere anreichern oder abtrennen und den rest nach Umisomerisierung wieder trennen.

Sowohl die reinen Isomeren als auch ihre Gemische werden von der Erfindung umfaßt.

Die Herstellung der Wirkstoffe erfolgt im allgemeinen dadurch, daß man dialkylsubstituierte Aniline mit Carbonylverbindungen wie beispielsweise Formaldehyd, Acetaldehyd, n-Butyraldehyd, Aceton, Methylethylketon, Diethylketon, 2-Ethylhexanal oder Cyclohexanon umsetzt.

Die Reaktion erfolgt in saurem Milieu, wobei neben Mineralsäuren auch saure Ionenaustauscher verwendet werden können. Besonders leicht reagieren die aromatischen Amine mit Formaldehyd bei 10 bis 100°C; hingegen reagieren Acetaldehyd, höhere aliphatische Aldehyde sowie aliphatische Ketone erheblich langsamer und nur bei höheren Temperaturen von etwa 100 bis 180°C, imsbesondere 150°C. In diesen Fällen ist es zweckmäßig, unter erhöhtem Druck (1 bis 20 bar) zu arbeiten.

Nach erfolgter Umsetzung setzt man die Amine mit Alkali frei und reinigt sie beispielsweise durch Destillation oder Kristallisation.

Anschließend werden die Amine nach bekannten Methoden zu den cycloalipphatischen Verbindungen hydriert und gegebenenfalls an den beiden Stickstoffatomen alkyliert.

Die Methylierung erfolgt am besten nach einer erprobten Methode mit Formaldehyd und Ameisensäure (H. T. Clarke et al, Am. Soc. 55, 4571 (1933).

Eine andere Herstellungsmöglichkeit der Wirkstoffe besteht darin, alkylierte Phenole mit Ketonen sauer zu Bisphenolen zu kondensieren, diese zu Biscyclohexanolderivaten zu hydrieren und anschließend mit Ammoniak oder niedrig substituierten Dialkylaminen und Wasserstoff an Hydrierkatalysatoren bei erhöhter Temperatur und erhöhtem Druck umzusetzen.

Der Zahl der Substituenten an den beiden Cyclohexanringen entsprechend erhält man eine große Zahl von Isomeren. Bei einer Verbindung, nämlich dem Bis-[3,5-diisopropyl-4-N-dimethyl-aminocyclohexyl]-methan, wurde eine Grobauftrennung mit Essigester in eine kristalline und eine nicht kristallisierende Fraktion durchgeführt.

Die Herstellung der Wirkstoffe sei an folgenden Beispielen erläutert:

**Beispiel 1**

655 Gew.-Teile 2,6-Diisopropylanilin wurden in 3 700 Gew.- Teilen Wasser und 400 Gew.-Teilen konzentrierter Salzsäure gelöst. Hierzu gab man im Verlauf einer Stunde 170 Gew.- Teile 30 %ige Formaldehydlösung in Wasser unter Rühren bei 92 bis 100°C hinzu. Nach weiteren 20 Min. setzte man durch Zugabe von 400 Gew.-Teilen 50 %iger Natronlauge bei 100°C die Base in Freiheit. Nach dem Abtrennen und Waschen destillierte man.

Sdp. bei 0,1 mb = 200 - 210°C. Ausbeute 605 Gew.-Teile = 97 % an Bis-[3,5-diisopropyl-4-aminophenyl]-methan.

Im einem Rührautoklaven gab man 600 Gew.-Teile Bis-(3,5-diisopropyl-4-amino-phenyl)-methan, 100 Gew.-Teile Dioxan und 2 Gew.-Teile Rutheniumoxidhydrat. Unter einem Druck von 300 bar Wasserstoff wurde bei einer Temperatur von 150 - 200°C hydriert. Nach dem Abfiltrieren des Katalysators destillierte man im Vakuum. Man erhielt 604 Gew.-Teile Bis-(3,5-di-isopropyl-4-aminocyclohexyl)-methan (Nr. 13) vom Siedepunkt 185°C bei einem Druck von 0,1 mbar. Dies entspricht einer Ausbeute von 97,5 %.

**Beispiel 2**

In einen Rührkolben wurden 1 460 Gew.-Teile Ameisensäure, 60 Gew.-Teile Wasser und 698 Gew.-Teile 30 %ige Formaldehydlösung vorgelegt. Hierzu ließ man unter Rühren 600 Gew.-Teile Bis-(3,5-diisopropyl-4-amino hexyl)-methan, in 400 Gew.-Teilen Dioxan gelöst, innerhalb von 10 Min. zulaufen. Die Temperatur stieg unter lebhafter Gasentwicklung bis auf 65°C an. Nach beendeter $CO_2$-Entwicklung dampfte man die überschüssige Ameisensäure ab, löst den Rückstand in Eiswasser, gab 200 Gew.-Teile Xylol hinzu und machte mit 850 Gew.-Teilen 50 prozentiger Natronlauge alkalisch. Man trennte die organische Phase ab und destillierte.

Sdp. bei 0,1 mbar = 200 - 210°C Ausbeute an Bis-[3,5-diisopropyl-4-N-dimethyl-amino-cyclohexyl]-methan 685 Gew.-Teile = 99,8 %.

Die Verbindung wurde durch Lösen und Abtrennen aus Essigsäureethylester in eine kristalline (Nr. 1) und eine nichtkristalline Fraktion (Nr. 2) getrennt.

In entsprechender Weise wurden folgende Verbindungen erhalten:

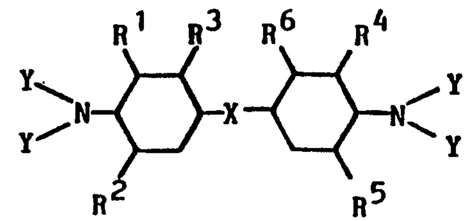

| Nr. | X | $-N<^{Y}_{Y}$ | $R^1$ $R^4$ | $R^2$ $R^5$ | $R^3$ $R^6$ | Sdp°C bei mb | |
|---|---|---|---|---|---|---|---|
| 1 | $CH_2$ | $N<^{Me}_{Me}$ | i-Prop | i-Prop | H | [FP 100 - 101°C] | |
| 2 | $CH_2$ | $N<^{Me}_{Me}$ | i-Prop | i-Prop | H | 200° | 0,1 |
| 3 | $CH_2$ | $NH_2$ | i-Prop | Me | H | 190° | 0,1 |
| 4 | $CH_2$ | $NH_2$ | Me | Me | H | 130° | 0,1 |
| 5 | $CH_2$ | $NH_2$ | i-Prop | Eth | H | 180° | 0,1 |
| 6 | $CH_2$ | $NH_2$ | t-But | Me | H | 170° | 0,2 |
| 7 | $CH_2$ | $NH_2$ | s-But | Eth | H | 180° | 0,1 |
| 8 | $CH_2$ | $N<^{Me}_{Me}$ | i-Prop | Eth | H | 170° | 0,1 |
| 9 | $CH_2$ | $N<^{Me}_{Me}$ | s-But | Eth | H | 175° | 0,1 |

| Nr. | X | $-N<^{Y}_{Y}$ | $R^1$ | $R^4$ | $R^2$ | $R^5$ | $R^3$ | $R^6$ | Sdp°C | bei mb |
|---|---|---|---|---|---|---|---|---|---|---|
| 10 | $CH_2$ | $N<^{Me}_{Me}$ | Me | | Me | | H | | 145° | 0,1 |
| 11 | $CH_2$ | $N<^{Me}_{Me}$ | | i-Prop | Me | | H | | 165° | 0,1 |
| 12 | $CH_2$ | $N<^{Me}_{Me}$ | | t-But | Me | | H | | 175° | 0,2 |
| 13 | $CH_2$ | $NH_2$ | | i-Prop | | i-Prop | H | | 185° | 0,1 |
| 14 | $CH_2$ | $N<^{Me}_{Me}$ | Me | | H | | H | | 130° | 0,1 |
| 15 | $CH_2$ | $N<^{Me}_{Me}$ | H | | H | | H | | 128° | 0,1 |
| 16 | $CH_2$ | $N<^{n-But}_{n-But}$ | H | | H | | H | | 200° | 0,1 |
| 17 | $CH_2$ | $N<^{Eth}_{Eth}$ | H | | H | | H | | 140° | 0,1 |
| 18 | $CH_2$ | $N<^{Eth}_{Eth}$ | H | | H | | Me | | 150° | 0,1 |
| 19 | – | $N<^{Me}_{Me}$ | Me | | Me | | H | | 125° | 0,1 |
| 20 | – | $NH_2$ | Me | | Me | | H | | 120° | 0,1 |
| 21 | – | $N<^{Me}_{Me}$ | H | | H | | H | | 100° | 0,1 |

| Nr. | X | $-N<^{Y}_{Y}$ | $R^1$ $R^4$ | $R^2$ $R^5$ | $R^3$ $R^6$ | Sdp°C | bei mb |
|---|---|---|---|---|---|---|---|
| 22 | $CH_2$ | $N<^{Eth}_{Eth}$ | s-But | Eth | H | 215° | 0,1 |
| 23 | $CH_2$ | $N<^{Me}_{Me}$ | OMe | H | OMe | 185° | 0,3 |
| 24 | $-CH_2-CH_2$ | $N<^{Me}_{Me}$ | H | H | H | 170° | 0,1 |
| 25 | $CMe_2$ | $NH_2$ | Me | Me | H | 160° | 0,1 |
| 26 | $CMe_2$ | $N<^{Me}_{Me}$ | Me | Me | H | 170° | 0,1 |
| 27 | $CH_2$ | $N<^{Me}_{Me}$ | $R^1 + R^3$ Tetramethylen | H | $R^4 + R^6$ Tetramethylen | 215° | 0,1 |
| 28 | $CH_2$ | $N<^{Me}_{Me}$ | i-Prop | H | H | 170° | 0,1 |
| 29 | $CMe_2$ | $NH_2$ | i-Prop | i-Prop | H | 190° | 0,1 |
| 30 | $CH_2$ | $N<^{Me}_{Me}$ | i-Prop | H | H | 170° | 0,1 |
| 31 | $CEth_2$ | $N<^{Me}_{Me}$ | Me | Me | H | 190° | 0,1 |
| 32 | $CMe_2$ | $N<^{Me}_{Me}$ | i-Prop | i-Prop | H | 220° | 0,1 |
| 33 | $CH_2$ | $N<^{Eth}_{Eth}$ | i-Prop | H | H | 170° | 0,1 |

| Nr. | X | $-N{<}^{Y}_{Y}$ | $R^1$ $R^4$ | $R^2$ $R^5$ | $R^3$ $R^6$ | Sdp°C bei mb |
|---|---|---|---|---|---|---|
| 34 | $CMe_2$ | $N{<}^{Me}_{Me}$ | s-But | Eth | H | 200° 0,1 |
| 35 | $CMe_2$ | $N{<}^{Me}_{Me}$ | Eth | Eth | H | 200° 0,1 |

Die neuen Wirkstoffe zeigen eine starke fungitoxische Wirksamkeit gegen phytopathogene Pilze. Sie dienen insbesondere zur Verhütung umd Heilung von Pflanzenkrankheiten, die durch Pilze der Klassen Phycomyceten und Deuteromyceten verursacht werden, wie beispielsweise:

Phytophthora infestans an Kartoffeln oder Tomaten
Phytophthora capsici an Paprika
Plasmopara viticola an Reben
Peronospora parasitica an Broccoli

Pseudoperonospora cubensis an Gurken oder Melonen
Pseudoperonospora humuli an Hopfen
Peronospora tabacina an Tabak
Botrytis cinerea an Reben oder Erdbeeren
Pyricularia oryzae an Reis
Pseudocerosporella herpotrichoides an Weizen, Gerste
Venturia imaequalis an Apfelbäumen
Alternaria solani an Tomaten, Kartoffeln
Cercospora spp.

Besonders interessant sind die neuen Fungizide für die Bekämpfung einer Vielzahl von Pilzen in verschiedenen Kulturen, insbesondere in Getreide, Obst- und Weinbau, Baumwolle, Soja sowie im Gemüse- und Zierpflanzenbau.

Die Wirkstoffe können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon, Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die Wirkstoffe können auch im Materialschutz u.a. zur Bekämpfung holzzerstörender Pilze wie Coniophora puteana und Polystictus versicolor eingesetzt werden. Die Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung Nr. 1 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung Nr. 5 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung Nr. 6 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 8 werden mit 3 Gewichtsteilen des Natriumsalzes der Diisobutylnaphthalin-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung Nr. 9 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 5 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 6 werden mit 2 Teilen Calciumsalz der Dodecylbenzolsulfonsäure, 8 Teilen

Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die Wirkstoffe dieser Erfindung kombiniert werden können, soll die Kombinationsmöglichkeiten erläutern, nicht aber einschränken:

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid
Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,
heterocyclische Strukturen, wie
2-Heptadecyl-2-imidazolin-acetat
2,4-Dichlor-6-(o-chloranilino)-s-triazin
0,0-Diethyl-phthalimidophosphonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethyl-phthalimid
N-Dichlorfluormethylthio-N',N'-dimethyl-N-phenyl-schwefelsäurediamid
5-Ethoxy-3-trichlotmethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilimo)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-l-H-1,2,4-triazol

N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-l-(lH-1,2,4-triazol-l-yl)-2-butanol
alpha-(2-Chlorphenyl)-alpha-(4-chlorphenyl)-5-pyrimidin-methanol
5-Butyl-2-dimethylamino-4-hyroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
sowie verschiedene Fungizide, wie
Dodecylguanidinacetat
3-(3-(3,5-Dimethyl-2-oxycyclohexyl)-2-hydroxyethyl)-gluataramid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl(-5-methyl-5-methoxymethyl-1,3-oxazolidin-2,4-dion
3-(3,5-Dichlorphenyl)-l-isopropylcarbamoylhydantoin
N-(3,5-Dichlorphenyl)-1,2-dimethylcyclopropan-1,2-dicarbonsäureimid.

Die folgenden Versuche erläutern die biologische Wirkung der Verbindungen. Vergleichsmittel (A) ist der bekannte Wirkstoff N-Trichlotmethylthio-tetrahydrophthalimid.

**Versuch 1**

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80 % Wirkstoff und 20 % Emulgiermittel in der Trockenmasse enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß beispielsweise die Verbindungen 2, 3, 4, 5, 6, 7, 8, 9, 10, 11, 13, 22, 28 und 29 bei der Anwendung als 0,05 %ige Spritzbrühe eine bessere fungizide Wirkung (z.B. 97 %) zeigen als der bekannte Wirkstoff A (z.B. 70 %);

**Versuch 2**

Wirksamkeit gegen Alternaria solani an Tomaten

Im Gewächshaus kultivierte Topfpflanzen der Sorte "Große Fleischtomate" werden im Vierblattstadium mit wäßriger Suspension besprüht, die aus der Trockensubstanz - bestehend aus 80 % Wirkstoff und 20 % Emulgator -aufbereitet wird. Nach dem Antrocknen des Spritzbelages werden die Blätter mit einer wäßrigen Sporensuspension des Pilzes Alternaria solani inoculiert. Die Pflanzen werden dann in eine wasserdampfgesättigte Kammer mit Temperaturen zwischen 22 und 24°C gestellt. Nach 4 Tagen hat sich die Krankheit auf den unbehandelten, jedoch inoculierten Pflanzen so stark entwickelt, daß die fungizide Wirksamkeit der Substanzen beurteilt werden kann.

Das Ergebnis des Versuches zeigt, daß beispielsweise die Verbindungen 1, 6, 9, 13 und 22 bei der Anwendung als 0,1 %ige Spritzbrühe eine gute fungizide Wirkung (z.B. 93 %) zeigen.

**Versuch 3**

Wirksamkeit gegen Phytophthora infestans an Tomaten

Blätter von Topfpflanzen der Sorte "Große Fleischtomate" werden mit wäßriger Spritzbrühe, die 80 % Wirkstoff und 20 % Emulgiermittel in der Trockenmasse enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Das Ergebnis des Versuches zeigt, daß beispielsweise die Verbindungen 1, 2, 3, 5, 6, 7, 8, 9, 13, 27, 28 und 29

bei der Anwendung als 0,025 %ige Spritzbrühe eine bessere fungizide Wirkung (z.B. 93 %) zeigen als der bekannte Wirkstoff A (z.B. 60 %).

**Versuch 4**

Wirksamkeit gegen Plasmopara viticola

Blätter vom Topfreben der Sorte "Müller-Thurgau" werden mit wäßriger Spritzbrühe, die 80 % Wirkstoff und 20 % Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Dann werden die Blätter mit einer Zoosporenaufschwemmung von Plasmopara viticola (Rebenperonospora) infiziert. Danach werden die Reben zunächst für 16 Stunden in einer einer wasser dampfgesättigten Kammer bei 24°C und anschließend für 8 Tage in einem Gewächshaus mit Temperaturen zwischen 20 und 30°C aufgestellt. Nach dieser Zeit werden die Pflanzen zur Beschleunigung des Sporangienträgerausbruches abermals für 16 Stunden in der feuchten Kammer aufgestellt. Dann erfolgt die Beurteilung des Ausmaßes der Pilzentwicklung auf den Blattunterseiten.

Das Ergebnis des Versuches zeigt, daß beispielsweise die Verbindungen 1, 2, 3, 5, 6, 8 und 13 bei der Anwendung als 0,025 %ige Spritzbrühe eine gute fungizide Wirkung (z.B. 97 %) zeigen.

**Patentansprüche** für die Vertragsstaaten BE CH FR GB IT LI NL SE

1. Biscyclohexylaminalkan der allgemeinen Formel

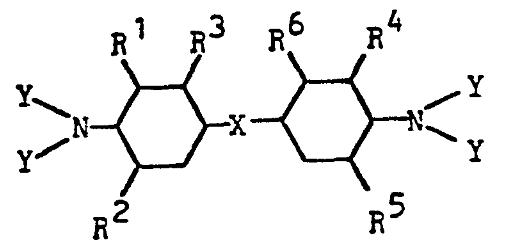

,

in der $R^1$, $R^2$, $R^4$ und $R^5$ gleich oder verschieden sind und Alkyl- oder Alkoxyreste mit 2 bis 5 C-Atomen je Rest, und $R^3$ und $R^6$ Wasserstoff bedeuten und $R^1$ und $R^3$ sowie $R^4$ und $R^6$ zusammen je einen an den Cyclohexylrest annellierten Cycloalkylrest bedeuten und $R^1$, $R^2$, $R^4$ und $R^5$ zusätzlich Wasserstoff bedeuten mit der Maßgabe, daß nur 2 der Reste $R^1$, $R^2$, $R^4$ und $R^5$ gleichzeitig Wasserstoff bedeuten, $R^3$ und $R^6$ zusätzlich Methoxy bedeuten mit der Maßgabe, daß 2 der Reste $R^1$, $R^2$, $R^4$ und $R^5$ Wasserstoff bedeuten,

X eine Einfachbindung, oder einen divalenten aliphatischen Rest mit bis 8 C-Atomen, oder einen Cyclohexylenrest bedeutet

und

Y einen Alkylrest mit einem bis vier C-atomen bedeutet, wobei zwei Reste Y zusammen mit dem N zu einem Ring geschlossen sein können, außer der Verbindung im der $R^1$, $R^2$, $R^4$ und $R^5$ Isopropyl, $R^3$ und $R^6$ Wasserstoff, X Methylen und Y Wasserstoff bedeutet.

2. Fungizid, enthaltend ein Biscyclohexylaminoalkan der allgemeinen Formel

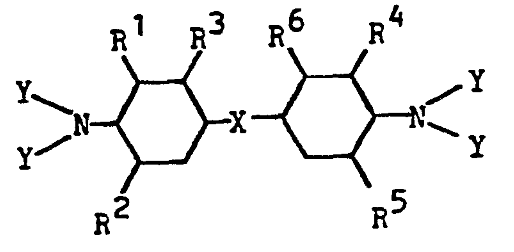

,

in der $R^1$, $R^2$, $R^4$ und $R^5$ gleich oder verschieden sind und Alkyl- oder Alkoxyreste mit 2 bis 5 C-Atomen je Rest, und $R^3$ und $R^6$ Wasserstoff bedeuten und $R^1$ und $R^3$ sowie $R^4$ und $R^6$ zusammen je einen an den Cyclohexylrest annellierten Cycloalkylrest bedeuten und $R^1$, $R^2$, $R^4$ und $R^5$ zusätzlich Wasserstoff bedeuten mit der Maßgabe, daß nur 2 der Reste $R^1$, $R^2$, $R^4$ und $R^5$ gleichzeitig Wasserstoff bedeuten, $R^3$ und $R^6$ zusätzlich

Methoxy bedeuten mit der Maßgabe daß 2 der Reste $R^1$, $R^2$, $R^4$ und $R^5$ Wasserstoff bedeuten,

X eine Einfachbindung, oder einen divalenten aliphatischen Rest mit 1 bis 8 C-Atomen, oder einen Cyclohexylenrest bedeutet

und

Y Wasserstoff oder einem Alkylrest mit einem bis vier C-Atomen bedeutet, wobei zwei Reste Y zusammen mit dem N zu einem Ring geschlossen sein können, außer der Verbindung in der $R^1$, $R^2$, $R^4$ und $R^5$ Isopropyl, $R^3$ und $R^6$ Wasserstoff, X Methylen und Y Wasserstoff bedeutet.

3. Fungizid, enthaltend einen festen oder flüssigen Trägerstoff und ein Biscyclohexylaminoalkan gemäß Anspruch 1.

4. Verfahren zur Herstellung eines Fungizids, dadurch gekennzeichnet, daß man einen festen oder flussigem Tragerstoff vermischt mit einem Biscyclohexylaminoalkan gemäß Anspruch 1.

5. Verfahren zur Bekämpfung von Pilzen, dadurch gekennzeichnet, daß man die Pilze oder die wor Pilzbefall zu schützenden Materialien, Pflanzen, Saatgüter oder den Boden mit einem Biscyclohexylaminalkan der folgenden allgemeinen Formel behandelt

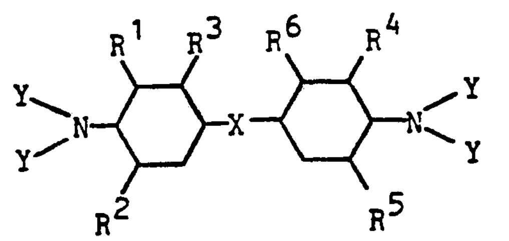

,

in der $R^1$, $R^2$, $R^3$, $R^4$, $R^5$ und $R^6$ gleich oder verschieden sind und Wasserstoff oder Alkyl- oder Alkoxyreste mit mit 1 bis 5 C-Atomen je Rest bedeuten, und $R^1$ und

$R^3$ sowie $R^4$ und $R^6$ zusammen je einen an den Cyclohexylrest annellierten Cycloalkylrest bedeuten,

X eine Einfachbindung oder einen divalenten aliphatischen Rest mit 1 bis 8 C-Atomen, oder einen Cyclohexylenrest bedeutet und Y Wasserstoff oder einen Alkylrest mit einem bis vier C-Atomen bedeutet, wobei zwei Reste Y zusammen mit dem N zu einem Ring geschlossen sein können.

**Patentansprüche** für den Vertragsstaat AT

1. Fungizid, enthaltend ein Biscyclohexylaminalkan der allgemeinen Formel

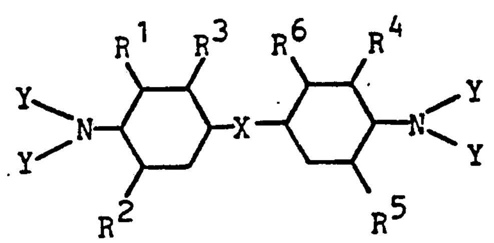

,

in der $R^1$, $R^2$, $R^4$ und $R^5$ gleich oder verschieden sind und Alkyl- oder Alkoxyreste mit 2 bis 5 C-Atomen je Rest, und $R^3$ und $R^6$ Wasserstoff bedeuten und $R^1$ und $R^3$ sowie $R^4$ und $R^6$ zusammen je einen an den Cyclohexylrest annellierten Cycloalkylrest bedeuten und $R^1$, $R^2$, $R^4$ und $R^5$ zusätzlich Wasserstoff bedeuten mit der Maßgabe, daß nur 2 der Reste $R^1$, $R^2$, $R^4$ und $R^5$ gleichzeitig Wasserstoff bedeuten, $R^3$ und $R^6$ zusätzlich Methoxy bedeuten mit der Maßgabe, daß 2 der Reste $R^1$, $R^2$, $R^4$ und $R^5$ Wasserstoff bedeuten,

X eine Einfachbindung, oder einen divalenten aliphatischen Rest mit 1 bis 8 C-Atomen, oder einen Cyclohexylenrest bedeutet

und

Y Wasserstoff oder einen Alkylrest mit einem bis vier C-Atomen bedeutet, wobei zwei Reste Y zusammen mit dem N zu einem Ring geschlossen sein können.

2. Fungizid, enthaltend einen festen oder flüssigen Trägerstoff und ein Biscyclohexylaminoalkan gemäß Anspruch 1.

3. Verfahren zur Herstellung eines Fungizids, dadurch gekennzeichnet, daß man einen festen oder flüssigen Trägerstoff vermischt mit einem Biscyclohexylaminoalkan gemäß Anspruch 1.

4. Verfahren zur Bekämpfung von Pilzen, dadurch gekennzeichnet, daß man die Pilze oder die vor Pilzbefall

zu schützenden Materialien, Pflanzen, Saatgüter oder den Boden mit einem Biscyclohexylaminalkan der folgenden allgemeinen Formel behandelt

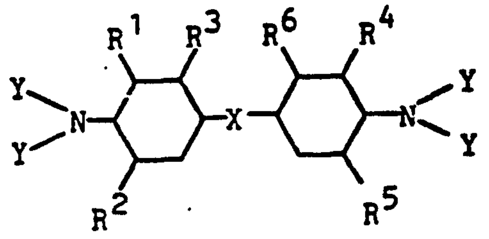

,

in der $R^1$, $R^2$, $R^3$, $R^4$, $R^5$ und $R^6$ gleich oder verschieden sind und Wasserstoff oder Alkyl- oder Alkoxyreste mit mit 1 bis 5 C-Atomen je Rest bedeuten, und $R^1$ und

$R^3$ sowie $R^4$ und $R^6$ zusammen je einen am den Cyclohexylrest annellierten Cycloalkylrest bedeuten,

X eine Einfachbindung oder einen divalenten aliphatischen Rest mit 1 bis 8 C-Atomen, oder einen Cyclohexylenrest bedeutet

und Y Wasserstoff oder einen Alkylrest mit einem bis vier C Atomem bedeutet, wobei zwei Reste Y zusammen mit dem N zu einem Ring geschlossen sein können.

**Claims** for the Contracting states: BE, CH, DE, FR, GB, IT, LI, NL, SE

1. A biscyclohexylamine-alkane of the general formula

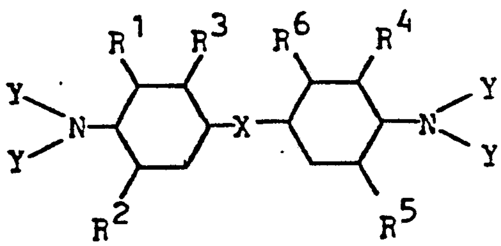

where

$R^1$, $R^2$, $R^4$ and $R^5$ are identical or different and are each alkyl or alkoxy, each of 2 to 5 carbon atoms, $R^3$ and $R^6$ are each hydrogen, $R^1$ and $R^3$ together and $R^4$ and $R^6$ together form a cycloalkyl radical fused to the cyclohexyl radical, and $R^1$, $R^2$, $R^4$ and $R^5$ may furthermore each be hydrogen, with the proviso that only two of the radicals $R^1$, $R^2$, $R^4$ and $R^5$ are simultaneously hydrogen, and $R^3$ and $R^6$ may furthermore each be methoxy, with the proviso that two of the radicals $R^1$. $R^2$, $R^4$ and $R^5$ are hydrogen,

X is a single bond, a divalent aliphatic radical of 1 to 8 carbon atoms or a cyclohexylene radical, and

Y is alkyl of 1 to 4 carbon atoms, and two radicals Y together with N can form a ring,

with the exception of the compound in which $R^1$, $R^2$, $R^4$ and $R^5$ are each isopropyl, $R^3$ and $R^6$ are each hydrogen, X is methylene and Y is hydrogen.

2. A fungicide containing a biscyclohexylamine-alkane of the general formula

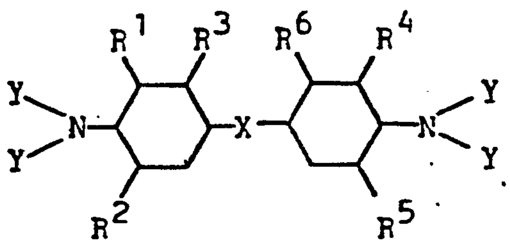

where

$R^1$, $R^2$, $R^4$ and $R^5$ are identical or different and are each alkyl or alkoxy, each of 2 to 5 carbon atoms, $R^3$ and $R^6$ are each hydrogen, $R^1$ and $R^3$ together and $R^4$ and $R^6$ together form a cycloalkyl radical fused to the cyclohexyl

radical, and $R^1$, $R^2$, $R^4$ and $R^5$ may furthermore each be hydrogen, with the proviso that only two of the radicals $R^1$, $R^2$, $R^4$ and $R^5$ are simultaneously hydrogen, and $R^3$ and $R^6$ may furthermore each be methoxy, with the proviso that two of the radicals $R^1$, $R^2$, $R^4$ and $R^5$ are hydrogen,

X is a single bond, a divalent aliphatic radical of 1 to 8 carbon atoms or a cyclohexylene radical, and

Y is hydrogen or alkyl of 1 to 4 carbon atoms, and two radicals Y together with N can form a ring,

with the exception of the compound in which $R^1$, $R^2$, $R^4$ and $R^5$ are each isopropyl, $R^3$ and $R^6$ are each hydrogen, X is methylene and Y is hydrogen.

3. A fungicide containing a solid or liquid carrier and a biscyclohexylamine-alkane as claimed in claim 1.

4. A process for the preparation of a fungicide, wherein a solid or liquid carrier is mixed with a biscyclohexylamine-alkane as claimed in claim 1.

5. A process for combatting fungi, wherein the fungi or the materials, plants, seed or soil to be protected against fungus attack are treated with a biscyclohexylamine-alkane of the following general formula

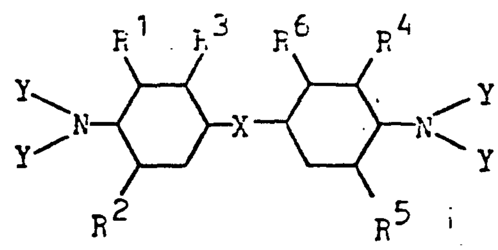

where

$R^1$, $R^2$, $R^3$, $R^4$, $R^5$ and $R^6$ are identical or different and are each hydrogen, or alkyl or alkoxy, each of 1 to 5 carbon atoms, or $R^1$ and $R^3$ together and $R^4$ and $R^6$ together form a cycloalkyl radical fused to the cyclohexyl radical,

X is a single bond, a divalent aliphatic radical of 1 to 8 carbon atoms or a cyclohexylene radical, and

Y is hydrogen or alkyl of 1 to 4 carbon atoms, and two radicals Y together with N can form a ring.

**Claims** For the Contracting state: AT

1. A fungicide containing a biscyclohexylamine-alkane of the general formula

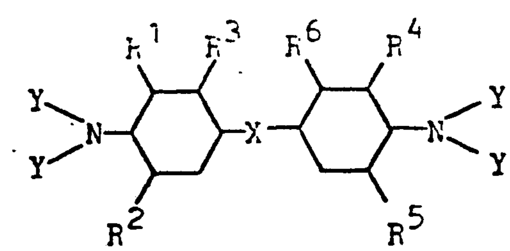

where

$R^1$, $R^2$, $R^4$ and $R^5$ are identical or different and are each alkyl or alkoxy, each of 2 to 5 carbon atoms, $R^3$ and $R^6$ are each hydrogen, $R^1$ and $R^3$ together and $R^4$ and $R^6$ together form a cycloalkyl radical fused to the cyclohexyl radical, and $R^1$, $R^2$, $R^4$ and $R^5$ may furthermore each be hydrogen, with the proviso that only two of the radicals $R^1$, $R^2$, $R^4$ and $R^5$ are simultaneously hydrogen, and $R^3$ and $R^6$ may furthermore each be methoxy, with the proviso that two of the radicals $R^1$, $R^2$, $R^4$ and $R^5$ are hydrogen,

X is a single bond, a divalent aliphatic radical of 1 to 8 carbon atoms or a cyclohexylene radical, and

Y is hydrogen or alkyl of 1 to 4 carbon atoms, and two radicals Y together with N can form a ring,

2. A fungicide containing a solid or liquid carrier and a biscyclohexylamine-alkane as defined in claim 1.

3. A process for the preparation of a fungicide, wherein a solid or liquid carrier is mixed with a biscyclohexylamine-alkane as defined in claim 1.

4. A process for combatting fungi, wherein the fungi or the materials, plants, seed or soil to be protected against fungus attack are treated with a biscyclohexylamine-alkane of the following general formula

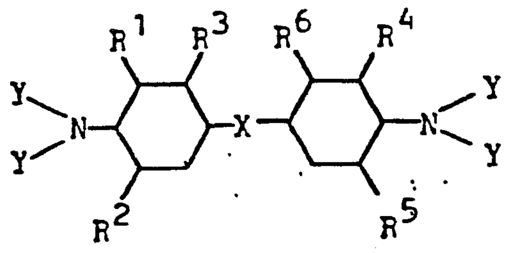

where

$R^1$, $R^2$, $R^3$, $R^4$, $R^5$ and $R^6$ are identical or different and are each hydrogen, or alkyl or alkoxy, each of 1 to 5 carbon atoms, or $R^1$ and $R^3$ together and $R^4$ and $R^6$ together form a cycloalkyl radical fused to the cyclohexyl radical,

X is a single bond, a divalent aliphatic radical of 1 to 8 carbon atoms or a cyclohexylene radical, and

Y is hydrogen or alkyl of 1 to 4 carbon atoms, and two radicals Y together with N can form a ring.

**Revendications** pour les Etats Contractants: BE, CH, DE, FR,

GB, IT, LI, NL, SE

1. Biscyclohexylaminalcane de formule générale

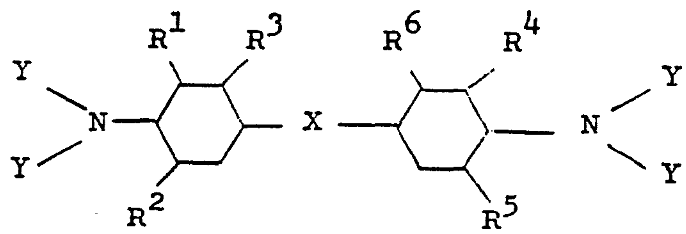

dans laquelle $R^1$, $R^2$, $R^4$ et $R^5$ sont identiques ou différents et représentent des restes alkyle ou alcoxy de 2 à 5 atomes C chacun et $R^3$ et $R^6$ de l'hydrogène et $R^1$ et $R^3$ ainsi que $R^4$ et $R^6$ représentent ensemble, chaque ensemble, un reste cycloalkyle condensé sur le reste cyclohexyle et $R^1$, $R^2$, $R^4$ et $R^5$ représentent, en outre, hydrogène, sous réserve que 2 seulement des restes $R^1$, $R^2$, $R^4$ et $R^5$ représentent simultanément hydrogène, $R^3$ et $R^6$ représentent en outre méthoxy sous réserve que 2 des restes $R^1$, $R^2$, $R^4$ et $R^5$ représentent hydrogène

X représente une liaison simple ou un reste aliphatique bivalent de 1 à 8 atomes C, ou un reste cyclohexylène et

Y représente un reste alkyle ayant un à quatre atomes C, deux restes Y pouvant, avec le N, se refermer en un cycle, excepte de composé dans lequel $R^1$, $R^2$, $R^4$ et $R^5$ représentent isopropyle, $R^3$ et $R^6$ hygrogène, X méthylène et Y hydrogène.

2. Fongicide contenant une biscyclohexylaminalcane de formule générale

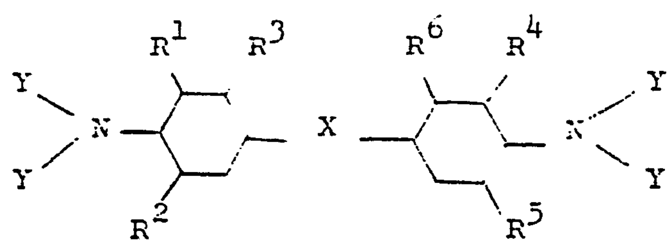

dans laquelle $R^1$, $R^2$, $R^4$ et $R^5$ sont identiques ou différents et représentent des restes alkyle ou alcoxy de 2 à 5 atomes C chacun et $R^3$ et $R^6$ de l'hydrogène et $R^1$ et $R^3$ ainsi que $R^4$ et $R^6$ représentent ensemble, chaque ensemble, un reste cycloalkyle condensé sur le reste cyclohexyle et $R^1$, $R^2$, $R^4$ et $R^5$ représentent, en outre, hydrogène, sous réserve que 2 seulement des restes $R^1$, $R^2$, $R^4$ et $R^5$ représentent simultanément hydrogène, $R^3$ et $R^6$ représentent en outre méthoxy sous réserve que 2 des restes $R^1$, $R^2$, $R^4$ et $R^5$ représentent hydrogène,

X représente une liaison simple ou un reste aliphatique bivalent de 1 à 8 atomes C, ou un reste cyclohexylène et

Y représente un reste alkyle ayant un à quatre atomes C, deux restes Y pouvant, avec le N, se refermer en un cycle, excepté le composé dans lequel $R^1$, $R^2$, $R^4$ et $R^5$ représentent isopropyle, $R^3$ et $R^6$ hydrogène, X méthyléne et Y hydrogène.

3. Fongicide contenant un véhicule solide ou liquide et une biscyclohexylaminoalcane selon la revendication 1.

4. procédé de préparation d'un fongicide, caractérisé par le fait que l'on mélange un véhicule solide ou liquide avec une biscyclohexylaminoalcane selon la revendication 1.

5. Procédé de lutte contre les champignons, caractérisé par le fait que l'on traite les champignons ou les matériaux, plantes, semences ou le sol à protéger d'une attaque de champignons, avec une biscyclohexylaminalcane de formule générale suivantè

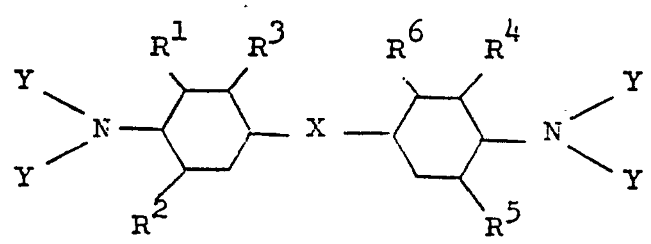

dans laquelle $R^1$ $R^2$ $R^3$ $R^4$ $R^5$ et $R^6$ sont identiques ou différents et représentent hydrogène ou des restes alkyle ou alcoxy de 1 à 5 atomes C chacun et $R^1$ et $R^3$ ainsi que $R^4$ et $R^6$, ensemble, representent respectivement un reste cycloalkyle condensé sur le reste cyclohexyle,

X représente une liaison simple ou un reste aliphatique bivalent de 1 à 8 atomes C, ou un reste cyclohexylène et

Y représente un reste alkyle ayant un à quatre atomes C, deux restes Y pouvant, avec le N, se refermer en un cycle.

**Revendications** pour l'Etat contractant: AT

1. Fongicide contouant une biscyclohexylaminalcane de formule générale

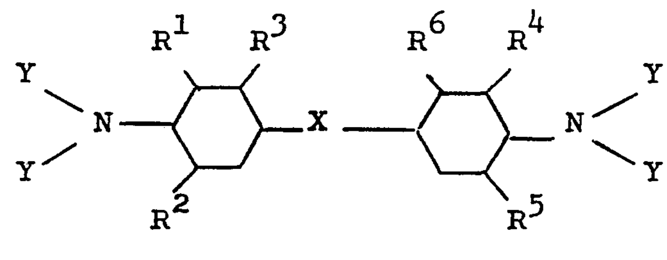

dans laquelle $R^1$, $R^2$, $R^4$ et $R^5$ sont identiques ou différents et représentent des restes alkyle ou alcoxy de 2 à 5 atomes C chacun et $R^3$ et $R^6$ de l'hydrogène et $R^1$ et $R^3$ ainsi que $R^4$ et $R^6$ représentent ensemble, chaque ensemble, un reste cycloalkyle condensé sur le reste cyclohexyle et $R^1$, $R^2$, $R^4$> et $R^5$ représentent, en outre, hydrogène, sous réserve que 2 seulement des restes $R^1$, $R^2$, $R^4$ et $R^5$ représentent simultanément hydrogène, $R^3$ et $R^6$ représentent en outre méthoxy sous réserve que 2 des restes $R^1$, $R^2$, $R^4$ et $R^5$ représentent hydrogène

X represente une liaison simple ou un reste aliphatique bivalent de 1 à 8 atomes C, ou un reste cyclohexylène et

Y représente un reste alkyle ayant un à quatre atomes C, deux restes Y pouvant avec le N, se refermer en un cycle,

2. Fongicide contenant un véhicule solide ou liquide et une biscyclohexylaminoalcane selon la revendication 1.

3. Procédé de préparation d'un fongicide, caractérisé par le fait que l'on mélange un véhicule solide ou liquide avec une biscyclohexylaminoalcane selon la revendication 1.

4. Procédé de lutte contre les champignons, caractérisé par le fait que l'on traite les champignons ou les matériaux, plantes, semences ou le sol à protéger d'une attaque de champignons, avec une biscyclohexylaminalcane de formule générale suivante

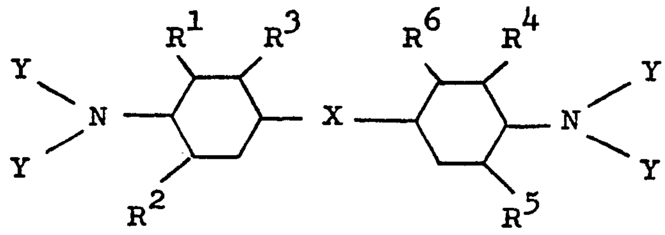

dans laquelle $R^1$, $R^2$, $R^3$, $R^4$, $R^5$ et $R^6$ sont identiques ou différents et représentent hydrogène ou des restes alkyle ou alcoxy de 1 à 5 atomes C chacun et $R^1$ et $R^3$ ainsi que $R^4$ et $R^6$, ensemble, représentent respectivement un reste cycloalkyle condensé sur le reste cyclohexyle,

X représente une liaison simple ou un reste aliphatique bivalent de 1 à 8 atomes C, ou un reste cyclohexylène et

Y représente un reste alkyle ayant un à quatre atomes C, deux restes Y pouvant, avec le N, se refermer en un cycle.